# EUROPÄISCHE PATENTSCHRIFT

(11) **EP 0 372 282 B1**
(45) Veröffentlichungstag und Bekanntmachung des Hinweises auf die Patenterteilung: **03.02.1993**
(21) Anmeldenummer: 89121375.3
(22) Anmeldetag: 18.11.1989
(51) Int. Cl.: C07C 211/58, C07C 209/18, C07C 37/04

(54) **Verfahren zur Herstellung von 1,5-Dihydroxy- und 1,5 Diaminonaphthalin**
Process for the preparation of 1,5-dihydroxy and 1,5 diaminonaphthalene
Procédé pour la préparation de 1,5-dihydro- et 1,5 diaminonaphthalène

(30) Priorität: 02.12.1988 DE 3840618
(43) Veröffentlichungstag der Anmeldung: 13.06.1990
(73) Patentinhaber: BAYER AG, 51368 Leverkusen (DE)
(72) Erfinder: Behre, Horst, Dr., D-5068 Odenthal-Eikamp (DE); Jakob, Lothar, Dr., D-8190 Wolfratshausen (DE); Blank, Heinz Ulrich, Dr., D-5068 Odenthal-Glöbusch (DE); Mayer, Dietmar, Dr., D-5060 Bergisch Gladbach 2 (DE); Busse, Roland, D-5093 Burscheid (DE)

(56) Entgegenhaltungen:
- EP-A- 0 085 883
- DE-C- 45 549
- DE-C- 45 788
- DE-C- 117 471
- ULLMANNS ENCYKLOPAEDIE DER TECHNISCHEN CHEMIE, Band 17, 4. Auflage, Seiten 85-126, Verlag Chemie Weinheim, New York
- HOUBEN-WEYL "METHODEN DER ORGANISCHEN CHEMIE", Band VI/1c, Teil 1, 1976, Seiten 208-210, Georg Thieme Verlag, Stuttgart
- CHEMICAL ABSTRACTS, Band 104, Nr. 5, 3. Februar 1986, Seite 527, Spalte 1, Zusammenfassung Nr. 33857d, Columbus, Ohio, US; & JP-A-60 35332

## Beschreibung

Die Erfindung betrifft ein verbessertes Verfahren zur Herstellung von 1,5-Diamino-naphthalin durch alkalische Druckhydrolyse des Dinatriumsalzes der Naphthalin-1,5-disulfonsäure und anschließende Aminierung des bei der Druckhydrolyse erhaltenen 1,5-Dihydroxy-naphthalins mit Ammoniak in Gegenwart von Ammoniumbisulfit zum 1,5-Diamino-naphthalin und von 1,5-Dihydroxy-naphthalin, dem in der 1. Stufe des Herstellungsverfahrens anfallenden Zwischenprodukt.

1,5-Dihydroxy- und 1,5-Diamino-naphthalin sind wichtige Zwischenprodukte für die Herstellung von Farbstoffen. 1,5-Diamino-naphthalin ist weiterhin ein wichtiges Zwischenprodukt für die Herstellung von Polyurethanen. Da für die Herstellung von Farbstoffen und die Weiterverarbeitung zum 1,5-Naphthylen-diisocyanat (→ Polyurethanen) ein hochreines 1,5-Diamino-naphthalin benötigt wird, hat sich die Fachwelt intensiv mit der Entwicklung von Ver fahren zur Herstellung von 1,5-Diamino-naphthalin befaßt, die das Produkt in guten Ausbeuten und der erforderlichen Reinheit liefern.

Für die technische Herstellung von 1,5-Diamino-naphthalin gibt es zwei verschiedene Verfahren:
A die Nitrierung von Naphthalin und nachfolgende Reduktion des Dinitronaphthalins;
B die Aminierung mit Ammoniak in Gegenwart von Ammoniumsulfit des durch Backschmelze aus dem Dinatriumsalz der Naphthalin-1,5-disulfonsäure erhaltenen 1,5-Dihydroxy-naphthalins (s. DE-PS 117 471; Ullmann's Encyklopädie der technischen Chemie, 4. Aufl. Bd. 17, S, 107/108).

Das Verfahren A hat den schwerwiegenden Nachteil, da bei der Nitrierung von Naphthalin ein Gemisch aus 1,8- und 1,5-Dinitronaphthalin entsteht, das beide Isomere im Verhältnis von etwa 2:1 enthält (siehe Houben-Weyl, 4. Aufl. Bd. 10/1, S. 494), daß es eine Auftrennung in die Isomeren erfordert, diese Isomerentrennung mit einem großen Aufwand und mit dem Zwangsanfall großer Mengen 1,8-Dinitronaphthalin verbunden ist.

Wegen der genannten Nachteile des Verfahrens A wird das Verfahren B in der Technik im allgemeinen bevorzugt. Das für die Herstellung von 1,5-Diamino-naphthalin nach Verfahren B benötigte 1,5-Dihydroxy-naphthalin wird bislang technisch durch Backschmelze, d.h. durch Verschmelzen von Naphthalin-1,5-disulfonsäure (Dinatriumsalz) mit Natriumhydroxid hergestellt. Es wurde zwar auch versucht die Backschmelze durch eine Druckschmelze, d.h. durch Erhitzen des Dinatriumsalzes der Naphthalin-1,5-disulfonsäure mit 50 %iger Natronlauge-Lösung unter Druck (= alkalische Druckhydrolyse) zu ersetzen. Von dieser Arbeitsweise wurde aber aus sicherheitstechnischen Gründen Abstand genommen, nachdem sich bei ihr verschiedentlich Unfälle ereignet hatten (siehe P.B. Report No. 74197, S. 54-55; Ullmanns Encyklopädie der technischen Chemie, 4. Aufl. Ed. 17, S. 87).

Es wurde nun gefunden, daß sich das Dinatriumsalz der Naphthalin-1,5-disulfonsäure gefahrlos durch Druckschmelze in das 1,5-Dihydroxy-naphthalin überführen läßt, wenn man bei der alkalischen Druckhydrolyse das Molverhältnis NaOH/Dinatriumsalz der Naphthalin-1,5-disulfonsäure wesentlich, nämlich von 7,8:1 auf mindestens 12:1 erhöht. Es wurde gefunden, daß die Druckhydrolyse bei Anwendung dieses NaOH-Überschusses nicht nur ohne sicherheitstechnisches Risiko durchführbar wird, sondern daß durch diese Maßnahme überraschenderweise auch die Qualität des erhaltenen 1,5-Dihydroxy-naphthalins wesentlich verbessert wird. Durch die alkalische Druckhydrolyse (Druckschmelze) unter Anwendung des erfindungsgemäßen Natriumhydroxid-Überschusses wird ein 1,5-Dihydroxy-naphthalin erhalten, dessen Gehalt an teerigen Bestandteilen (höhermolekulare, in Chlorbenzol unlösliche Nebenprodukte) auf einen Bruchteil des Gehal tes an teerigen Bestandteilen sinkt, den das durch Backschmelze oder durch Druckschmelze mit üblichen Mengen Natriumhydroxid erhaltene 1,5-Dihydroxy-naphthalin aufweist. Diese teerigen Bestandteile werden bei der Aminierung des 1,5-Dihydroxy-naphthalins zum 1,5-Diamino-naphthalin weitergeschleppt und müssen bislang aus diesem durch aufwendige Reinigungsverfahren, z.B. Sublimation des Roh-1,5-diaminonaphthalins, abgetrennt werden. Bei Verwendung des durch Druckhydrolyse mit dem erfindungsgemäßen Natriumhydroxid-Überschuß hergestellten 1,5-Dihydroxy-naphthalins wird ein 1,5-Diamino-naphthalin erhalten, das einen so geringen Gehalt an teerigen Bestandteilen aufweist, daß es vor seiner Weiterverarbeitung, z.B. zum Naphthylen-1,5-diisocyanat, keiner Reinigung mehr bedarf.

Die Erfindung betrifft daher ein Verfahren zur Herstellung von 1,5-Diamino-naphthalin durch alkalische Druckhydrolyse des Dinatriumsalzes der Naphthalin-1,5-disulfonsäure mit überschüssigem Natriumhydroxid bei Temperaturen von 270 bis 290°C und Drucken von 14 bis 20 bar und Aminieren des bei der Druckhydrolyse erhaltenen 1,5-Dihydroxy-naphthalins mit Ammoniak in Gegenwart von Ammoniumbisulfit, das dadurch gekennzeichnet ist, daß man das Natriumhydroxid für die alkalische Druckhydrolyse in einem solchen Überschuß anwendet, daß das Molverhältnis NaOH/Dinatriumsalz der Naphthalin-1,5-disulfonsäure mindestens 12:1, vorzugsweise 14:1 bis 18:1, besonders bevorzugt 15:1 bis 17:1 beträgt.

Die Erfindung betrifft gleichzeitig aber auch das Verfahren zur Herstellung von 1,5-Dihydroxy-naphthalin durch alkalische Druckhydrolyse des Dinatriumsalzes der Naphthalin-1,5-disulfonsäure mit überschüssigem Natriumhydroxid bei Temperaturen von 270 bis 290°C und Drucken von 14 bis 20 bar, das dadurch gekennzeichnet ist, daß das Natriumhydroxid für die alkalische Druckhydrolyse in einem solchen Überschuß angewendet wird, daß das Molverhältnis NaOH/Dinatriumsalz der Naphthalin-1,5-disulfonsäure mindestens 12:1, vorzugsweise 14:1 bis 18:1, besonders bevorzugt 15:1 bis 17:1 beträgt.

Ferner wurde gefunden, daß sich die Qualität des 1,5-Diamino-naphthalins weiterhin durch folgende neue Maßnahmen bei der Aminierung des 1,5-Dihydroxy-naphthalins mit Ammoniak in Gegenwart von Ammoniumhydrogensulfit verbessern läßt:
a) bei der Aminierung mit einem größeren Ammoniak-Überschuß zu arbeiten als bislang und Ammoniak und 1,5-Dihydroxy-naphthalin in Molverhältnissen von mindestens 6:1, vorzugsweise 6,5:1 bis 12:1, besonders bevorzugt 7:1 bis 9:1 einzusetzen; und - vor allem -
b) das Reaktionsgemisch nach beendeter Aminierungsreaktion bei Temperaturen von mindestens 145°C, vorzugsweise 150 bis 180°C, mit mindestens 1,5 Mol, vorzugsweise 1,55 bis 2 Mol, besonders bevorzugt 1,6 bis 1,8 Mol Natriumhydroxid je Mol eingesetzten 1,5-Dihydroxy-naphthalins zu versetzen.

Diese beiden neuen Maßnahmen bei der Aminierung bewirken, daß ein 1,5-Diamino-naphthalin erhalten wird, das praktisch frei ist von dem seine Weiterverarbeitung empfindlich störenden 1-Amino-5-hydroxy-naphthalin.

Die Erfindung betrifft daher bevorzugt ein Verfahren zur Herstellung von 1,5-Diamino-naphthalin durch alkalische Druckhydrolyse des Dinatriumsalzes der Naphthalin-1,5-disulfonsäure mit überschüssigem Natriumhydroxid bei Temperaturen von 270 bis 290°C und Drucken von 14 bis 20 bar und Aminieren des bei der alkalischen Druckhydrolyse erhaltenen 1,5-Dihydroxy-naphthalins mit Ammoniak in Gegenwart von Ammoniumhydrogensulfit bei erhöhtem Druck und erhöhter Temperatur, das dadurch gekennzeichnet ist,
a) daß man das Natriumhydroxid für die alkalische Druckhydrolyse in einem solchen Überschuß anwendet, daß das Molverhältnis NaOH:Dinatriumsalz der Naphthalin-1,5-disulfonsäure mindestens 12:1, vorzugsweise 14:1 bis 18:1, besonders bevorzugt 15:1 bis 17:1 beträgt;
b) daß man die Aminierung mit einem solchen Ammoniak-Überschuß vornimmt, daß das Molverhältnis Ammoniak/1,5-Dihydroxy-naphthalin mindestens 6:1, vorzugsweise 6,5:1 bis 12:1, besonders bevorzugt 7:1 bis 9:1 beträgt; und
c) daß man das Reaktionsgemisch nach beendeter Aminierungsreaktion bei Temperaturen von mindestens 145°C, vorzugsweise 150 bis 180°C, mit mindestens 1,5 Mol, vorzugsweise 1,55 bis 2 Mol, besonders bevorzugt 1,6 bis 1,8 Mol NaOH je Mol eingesetzten 1,5-Dihydroxy-naphthalins versetzt.

Das für die alkalische Druckhydrolyse benötigte Dinatriumsalz der Naphthalin-1,5-disulfonsäure kann, statt es als solches einzusetzen, auch im Hydrolysegemisch aus der freien Naphthalin-1,5-disulfonsäure bzw. deren Tetrahydrat und einer für die Neutralisation der freien Säure erforderlichen Menge Natriumhydroxid in situ erzeugt werden.

Die Aufarbeitung des Hydrolysegemisches erfolgt in an sich bekannter Weise, beispielsweise durch Verdünnen der alkalischen Reaktionsmischung mit Wasser, Neutralisation der alkalischen Lösung mit Mineralsäure, beispielsweise Salzsäure oder Schwefelsäure, und mechanisches Abtrennen (durch Filtrieren oder Abschleudern) des ausgefallenen 1,5-Dihydroxy-naphthalins.

Das Aufarbeiten des nach der Natriumhydroxid-Zugabe vorliegenden Aminierungsgemisches und die Isolierung des 1,5-Diamino-naphthalins erfolgt ebenfalls in an sich bekannter Weise, beispielsweise durch Abdestillieren des nicht umgesetzten Ammoniaks in Form einer wäßrigen Ammoniaklösung aus dem Reaktionsgemisch und Abtrennen (Abfiltrieren bzw. Abzentrifugieren) des ausgefallenen 1,5-Diamino-naphthalins.

Die Qualität des nach dem erfindungsgemäßen Verfahren erhaltenen 1,5-Diamino-naphthalins entspricht der von gereinigtem 1,5-Diamino-naphthalin. Das erfindungsgemäß erhaltene 1,5-Diamino-naphthalin kann deshalb ohne weitere Reinigung durch Phosgenierung in hohen Ausbeuten zu Naphthalin-1,5-diisocyanat umgesetzt werden. Da das erfindungsgemäß erhaltene 1,5-Diamino-naphthalin keine teerigen Anteile enthält, enthält auch das aus ihm hergestellte Naphthalin-1,5-diisocyanat keine teerigen Nebenprodukte, die bei der Herstellung des Diisocyanates zu Schwierigkeiten führen könnten

### Beispiel 1

Eine Mischung aus 374 g (= 1,0 Mol) Naphthalin-1,5-disulfonsäure (Dinatriumsalz) (89 gew.-%iges Produkt), 600 g Natriumhydroxid (15,0 Mol) und 237 g Wasser wird in einem 1,3-Liter-Nickel-Autoklaven innerhalb von 2,5 bis 3 Stunden unter Rühren auf 285°C erhitzt und 2 Stunden auf dieser Temperatur gehalten. Nach dem Abkühlen des Autoklaveninhalts auf etwa 120°C werden 400 g Wasser eingepumpt. Das etwa 80 bis 90°C warme Gemisch wird aus dem Autoklaven in ein anderes Reaktionsgefäß überführt und dort mit etwa 1200 g Wasser auf ein Gesamtgewicht von etwa 2800 g verdünnt. Die verdünnte Reaktionsmischung wird bei 80°C innerhalb einer Stunde gleichzeitig mit 1470 g 50 gew.-%iger Schwefelsäure in 500 g Wasser unter Rühren eingetragen. Die erhaltene Mischung wird 1 Stunde bei 80°C nachgerührt, im Verlauf von 2 Stunden auf 60°C abgekühlt und abschließend 1 Stunde bei 60°C gerührt. Das 1,5-Dihydroxy-naphthalin wird bei 60°C abfiltriert, dreimal mit je 500 g warmem Wasser (60°C) gewaschen und abschließend im Vakuum getrocknet.

Es werden 158 g (= 96,6 % der Theorie, bezogen auf eingesetzte Naphthalin-1,5-disulfonsäure) erhalten.

### Zusammensetzung:

98,1 Gew.-% 1,5-Dihydroxy-naphthalin
0 bis 0,1 Gew.-% 5-Hydroxy-naphthalin-1-sulfonsäure
0,1 bis 0,5 Gew.-% 1-Naphthol
Rest bis 100 Gew.-%: Wasser, Na₂SO₄, Na₂SO₃, unbekannte Nebenprodukte.

Ein Gemisch aus 130 g 1,5-Dihydroxy-naphthalin (98,1 gew.-%ig = 0,8 Mol), 102 g Ammoniak (6,0 Mol), 52 ml Ammoniumhydrogensulfitlösung (490 g SO₂ je Liter = 0,4 Mol) und 449 g Wasser wird in einem 1,3-Liter-Titan-Autoklaven unter Rühren auf 155°C erhitzt und 4 Stunden bei dieser Temperatur gerührt. Anschließend werden unter Rühren bei 155°C 102 g 50 gew.-%ige Natronlauge (1,28 Mol NaOH) eingepumpt. Das Reaktionsgemisch wird auf 70°C abgekühlt, und 1 Stunde bei dieser Temperatur nachgerührt. Anschließend wird das ausgefallene 1,5-Diamino-naphthalin abfiltriert und mehrmals mit 70°C heißem Wasser gewaschen. Nach dem Trocknen im Vakuum werden 110 g 1,5-Diamino-naphthalin (= 86 % der Theorie, bezogen auf eingesetztes 1,5-Dihydroxy-naphthalin) erhalten.

### Zusammensetzung:

99,0 Gew.-% 1,5-Diamino-naphthalin
0,2 Gew.-% 1-Amino-naphthalin
0,0-0,05 Gew.-% 1-Amino-5-hydroxynaphthalin
0,4 Gew.-% niedermolekulare Nebenprodukte
0,1 Gew.-% Asche
0,1 Gew.-% Wasser
0,2 Gew.-% Chlorbenzol - unlösliche Nebenprodukte

### Beispiel 2

Eine Mischung aus 369,2 g (= 1,0 Mol) Naphthalin-1,5-disulfonsäure (Tetrahydrat) (97,5 gew.-%iges Produkt) 680 g Natriumhydroxid (17,0 Mol) und 165 g Wasser wird in einem 1,3-Liter-Nickel-Autoklaven innerhalb von 2,5 bis 3 Stunden unter Rühren auf 285°C erhitzt und 2 Stunden bei dieser Temperatur gehalten. Nach dem Abkühlen des Autoklaven-Inhaltes auf etwa 120°C werden 400 g Wasser eingepumpt. Der Autoklaveninhalt wird mit einer Temperatur von 80°C bis 90°C in ein offenes Reaktionsgefäß überführt und dort mit 1200 g Wasser auf ein Gesamtgewicht von etwa 2800 g verdünnt. Die verdünnte Reaktionsmischung wird bei 80°C innerhalb 1 Stunde gleichzeitig mit 1470 g 50 gew.-%iger Schwefelsäure in 500 g Wasser eingetragen; das Gemisch wird 1 Stunde bei 80°C gerührt, dann innerhalb von 2 Stunden auf 60°C abgekühlt und 1 Stunde bei 60°C gerührt. Das 1,5-Dihydroxy-naphthalin wird bei 60°C abfiltriert, dreimal mit je 500 g warmem Wasser (60°C) gewaschen und im Vakuum getrocknet. Es werden 158 g (96,6 % der Theorie, bezogen auf eingesetzte Naphthalin-1,5-disulfonsäure) erhalten.

### Zusammensetzung:

98,1 Gew.-% 1,5-Dihydroxy-naphthalin
0 bis 0,1 Gew.-% 5-Hydroxy-naphthalin-1-sulfonsäure
0,1 bis 0,5 Gew.-% 1-Naphthol

Ein Gemisch aus 130,5 g 1,5-Dihydroxy-naphthalin (98,1 gew.-%ig = 0,8 Mol), 136 g Ammoniak (8,0 Mol), 52 ml Ammoniumhydrogensulfitlösung (490 g SO₂ je Liter = 0,4 Mol) und 449 g Wasser werden in einem 1,3-Liter-Titan-Autoklaven unter Rühren auf 155°C erhitzt und 4 Stunden bei dieser Temperatur gerührt. Anschließend werden bei gleicher Temperatur 140 g 50 gew.-%ige Natronlauge (1,75 Mol NaOH) eingepumpt. Das Reaktionsgemisch wird auf 70°C abgekühlt und 1 Stunde bei dieser Temperatur nachgerührt. Anschließend wird das 1,5-Diamino-naphthalin abfiltriert, mehrmals mit 70°C heißem Wasser gewaschen und abschließend im Vakuum getrocknet.

Es werden 110 g 1,5-Diamino-naphthalin (86 % der Theorie, bezogen auf eingesetztes 1,5-Dihydroxy-naphthalin) erhalten.

### Zusammensetzung:

99 Gew.-% 1,5-Diamino-naphthalin
0,2 Gew.-% 1-Aminonaphthalin
0,0 Gew.-% 1-Amino-5-hydroxy-naphthalin
0,3 Gew.-% niedermolekulare Nebenprodukte
0,1 Gew.-% Asche
0,1 Gew.-% Wasser
0,3 Gew.-% Chlorbenzol - unlösliche Nebenprodukte

## Patentansprüche

1. Verfahren zur Herstellung von 1,5-Diamino-naphthalin durch alkalische Druckhydrolyse des Dinatriumsalzes der Naphthalin-1,5-disulfonsäure mit überschüssigem Natriumhydroxid bei Temperaturen von 270 bis 290°C und Drucken von 14 bis 20 bar und Aminieren des bei der Druckhydrolyse erhaltenen 1,5-Dihydroxy-naphthalins mit Ammoniak in Gegenwart von Ammoniumbisulfit, dadurch gekennzeichnet, daß man das Natriumhydroxid für die alkalische Druckhydrolyse in einem solchen Überschuß anwendet, daß das Molverhältnis NaOH/Dinatriumsalz der Naphthalin-1,5-disulfonsäure mindestens 12:1 beträgt.

2. Verfahren gemäß Anspruch 1, dadurch gekennzeichnet, daß das Molverhältnis NaOH/Dinatriumsalz der Naphthalin-1,5-disulfonsäure 14:1 bis 18:1 beträgt.

3. Verfahren gemäß Anspruch 1 oder 2, dadurch gekennzeichnet,
daß man die Aminierung mit einem solchen Ammoniak-Überschuß vornimmt, daß das Molverhältnis Ammoniak/1,5-Dihydroxy-naphthalin mindestens 6:1 beträgt und
daß man das Reaktionsgemisch nach beendeter Aminierungsreaktion bei Temperaturen von mindestens 145°C mit mindestens 1,5 Mol NaOH je Mol eingesetzten 1,5-Dihydroxy-naphthalins versetzt.

4. Verfahren gemäß Anspruch 3, dadurch gekennzeichnet, daß das Molverhältnis Ammoniak/1,5-Dihydroxy-naphthalin 6,5:1 bis 12:1 beträgt und daß man das Reaktionsgemisch bei Temperaturen von 150 bis 180°C mit 1,55 bis 2 Mol NaOH je Mol eingesetzten 1,5-Dihydroxy-naphthalins versetzt.

5. Verfahren zur Herstellung von 1,5-Dihydroxy-naphthalin durch alkalische Druckhydrolyse des Dinatriumsalzes der Naphthalin-1,5-disulfonsäure mit überschüssigem Natriumhydroxid bei Temperaturen von 270 bis 290°C und Drucken von 14 bis 20 bar, dadurch gekennzeichnet, daß das Natriumhydroxid für die alkalische Druckhydrolyse in einem solchen Überschuß angewendet wird, daß das Molverhältnis NaOH/Dinatriumsalz der Naphthalin-1,5-disulfonsäure mindestens 12:1 beträgt.

## Claims

1. Process for the preparation of 1,5-diaminonaphthalene by alkaline pressure hydrolysis of the disodium salt of naphthalene-1,5-disulphonic acid with excess sodium hydroxide at temperatures from 270 to 290°C and under pressures from 14 to 20 bar and by amination with ammonia in the presence of ammonium bisulphite of the 1,5-dihydroxynaphthalene obtained in the pressure hydrolysis, characterized in that the sodium hydroxide for the alkaline pressure hydrolysis is used in an excess such that the molar ratio NaOH/disodium salt of naphthalene-1,5-disulphonic acid is at least 12:1.

2. Process according to Claim 1, characterized in that the molar ratio NaOH/disodium salt of naphthalene-1,5-disulphonic acid is 14:1 to 18:1.

3. Process according to Claim 1 or 2, characterized in that the amination is carried out with an excess of ammonia such that the molar ratio ammonia/1,5-dihydroxynaphthalene is at least 6:1 and that, when the amination reaction is complete, at least 1.5 mol of NaOH per mol of 1,5-dihydroxynaphthalene employed is added to the reaction mixture at temperatures of at least 145°C.

4. Process according to Claim 3, characterized in that the molar ratio ammonia/1,5-dihydroxynaphthalene is 6.5:1 to 12:1 and that 1.55 to 2 mol of NaOH per mol of 1,5-dihydroxynaphthalene employed are added to the reaction mixture at temperatures from 150 to 180°C.

5. Process for the preparation of 1,5-dihydroxynaphthalene by alkaline pressure hydrolysis of the disodium salt of naphthalene-1,5-disulphonic acid with excess sodium hydroxide at temperatures from 270 to 290°C and under pressures from 14 to 20 bar, characterized in that the sodium hydroxide for the alkaline pressure hydrolysis is used in an excess such that the molar ratio NaOH/disodium salt of naphthalene-1,5-disulphonic acid is at least 12:1.

## Revendications

1. Procédé de préparation du 1,5-diaminonaphtalène par hydrolyse alcaline sous pression du sel disodique de l'acide naphtalène-1,5-disulfonique par un excès d'hydroxyde de sodium à des températures de 270 à 290°C et des pressions de 14 à 20 bar et amination par l'ammoniac en présence de bisulfite d'ammonium du 1,5-dihydroxynaphtalène obtenu à l'hydrolyse sous pression, caractérisé en ce que l'on utilise l'hydroxyde de sodium nécessaire pour l'hydrolyse alcaline sous pression en excès tel que le rapport molaire NaOH/sel disodique de l'acide naphtalène-1,5-disulfonique soit d'au moins 12:1.

2. Procédé selon la revendication 1, caractérisé en ce que le rapport molaire NaOH/sel disodique de l'acide naphtalène-1,5-disulfonique va de 14:1 à 18:1.

3. Procédé selon la revendication 1 ou 2, caractérisé en ce que :
on procède à l'amination avec un excès d'ammoniac tel que le rapport molaire ammoniac/1,5-dihydroxy naphtalène soit d'au moins 6:1, et
après la réaction d'amination, on ajoute au mélange de réaction, à des températures d'au moins 145°C, au moins 1,5 mol de NaOH par mole de 1,5-dihydroxynaphtalène mis en oeuvre.

4. Procédé selon la revendication 3, caractérisé en ce que le rapport molaire ammoniac/1,5-dihydroxynaphtalène va de 6,5:1 à 12:1 et en ce que l'on ajoute au mélange de réaction, à des températures de 150 à 180°C, 1,55 à 2 mol de NaOH par mole de 1,5-dihydroxynaphtalène mis en oeuvre.

5. Procédé de préparation du 1,5-dihydroxynaphtalène par hydrolyse alcaline sous pression du sel disodique de l'acide naphtalène-1,5-disulfonique par un excès d'hydroxyde de sodium à des températures de 270 à 290°C et des pressions de 14 à 20 bar, caractérisé en ce que l'on utilise l'hydroxyde de sodium nécessaire pour l'hydrolyse alcaline sous pression en excès tel que le rapport molaire NaOH/sel disodique de l'acide naphtalène-1,5-disulfonique soit d'au moins 12:1.
